Europäisches Patentamt

European Patent Office

Office européen des brevets

(11) Publication number: **0 083 375**
**B1**

# EUROPEAN PATENT SPECIFICATION

(12)

(45) Date of publication of patent specification: **20.05.87**

(21) Application number: **82902088.2**

(22) Date of filing: **07.07.82**

(86) International application number:
**PCT/JP82/00255**

(87) International publication number:
**WO 83/00148 20.01.83 Gazette 83/02**

(51) Int. Cl.⁴: **C 07 G 11/00,** C 12 P 1/04,
A 61 K 35/74

(54) **ANTIBACTERIAL EFFECT-ENHANCING SUBSTANCE AND PROCESS FOR ITS PREPARATION.**

(30) Priority: **07.07.81 JP 106554/81**

(43) Date of publication of application:
**13.07.83 Bulletin 83/28**

(45) Publication of the grant of the patent:
**20.05.87 Bulletin 87/21**

(84) Designated Contracting States:
**BE CH DE FR GB LI NL SE**

(56) References cited:
EP-A-0 086 610
GB-A-2 011 380
US-A-4 225 586

Aida Hiroshi "Oyo Biseibutsu-gaku", 1. March,
1979(01.03.79), Dobun Shoin, pages 188-203

(73) Proprietor: **Takeda Chemical Industries, Ltd.**
**27, Doshomachi 2-chome Higashi-ku**
**Osaka-shi Osaka, 541 (JP)**

(72) Inventor: **IMADA, Akira**
**18-16, Kurakuen 4-bancho Nishinomiya-shi**
**Hyogo 662 (JP)**
Inventor: **KINTAKA, Kazuhiko**
**21-9, Ushitora 2-chome Ibaraki-shi**
**Osaka 567 (JP)**
Inventor: **SHINAGAWA, Susumu**
**3-31, Higashiishikiricho 6-chome Higashiosaka-shi**
**Osaka 579 (JP)**

(74) Representative: **Laredo, Jack Joseph et al**
**Elkington and Fife High Holborn House 52/54**
**High Holborn**
**London, WC1V 6SH (GB)**

Courier Press, Leamington Spa, England.

# 0 083 375

**Description**

Technical field

This invention relates to a novel substance F2 which potentiates the activity of antibiotics, salts thereof, and methods of producing F2 and said salts.

Background art

Until now, the protein SP127 has been known as a substance which potentiates the activity of antibiotics (M. Kikuchi et al.; The Journal of Antibiotics *30*, 209—214, 1977), and it potentiates the antibacterial activity of macrolide antibiotics (ibid. *30,* 215—220, 1977). On the other hand, clavulanic acid is known as a substance which prevents inactivation of β-lactam antibiotics by β-lactamases and synergistically potentiates its activity against β-lactamase producing bacteria [D. A. Leigh et al., Journal of Antimicrobial Chemotherapy, (1981)7, 229—236].

Disclosure of the invention

This invention relates to a substance F2 which potentiates the activity of antibiotics, salts thereof, and a method of producing them.

Namely, this invention relates, in one aspect, to (1) a substance of F2 which potentiates the activity of antibiotics against gram-negative bacteria, inclusive of salts thereof and salts produced synthetically therefrom, which as a monosodium salt thereof has the following physical and chemical properties:

(1) Appearance: Colorless crystalline powder
(2) Elemental analysis (%):

|   |   |
|---|---|
| C | 31.81±1.0 |
| H | 5.35±0.5 |
| N | 6.96±0.5 |
| S | 10.81±1.0 |
| Na | 3.87±0.5 |

(3) Ultraviolet absorption spectrum:
No characteristic absorption at wavelength over 210 nm.
(4) Infrared absorption spectrum:
Main absorptions (wave numbers) in the IR region of the spectrum (KBr disc): 1690, 1665, 1555, 1375, 1220, 1050, 820 $(cm^{-1})$.
(5) Specific rotation:
$[\alpha]_D^{26}+6°±2°$ $(N—CH_3COOH)$
(6) Solubility:
Insoluble in petroleum ether, hexane, diethyl ether, benzene, ethyl acetate and chloroform; sparingly soluble in ethanol, pyridine and acetone; soluble in methanol and dimethyl sulfoxide; readily soluble in water.
(7) Basic, neutral or acidic:
Acidic
(8) Molecular weight:
594±77 (as monosodium salt, calcd. from sodium content)
(9) Color reactions:
Positive Greig-Leaback and potassium permanganate reactions; negative ninhydrin, Sakaguchi and Molisch reactions,

in another aspect, to (2) a method of producing a substance F2 as defined above characterised by cultivating in a culture medium the strain Pseudomonas acidophila (FERM-P 4344, IFO 13774, ATCC 31363) or Pseudomonas mesoacidophila (FERM-P 4653, IFO 13884, ATCC 31433) which is capable of producing the substance F2, and harvesting from the resulting culture broth the substance F2 thus produced as a metabolite, and in a further aspect to (3) the use of a substance F2 as defined above in association with a β-lactam antibiotic to make a therapeutic composition for the treatment of infectious diseases caused by gram-negative bacteria in mammals.

The present inventors isolated from soil samples a large number of microorganisms for the purpose of developing new antibiotic substances and searched for antibiotics in the metabolites elaborated by these microorganisms. This exploration led them to the finding that certain strains among these isolates would produce a novel substance which potentiates antibacterial activity, that the strains belong to the genus *Pseudomonas,* and that a substance capable of potentiating the antibacterial activity of β-lactam antibiotics against gram-negative bacteria can be caused to accumulate in a culture medium by growing such a strain of microorganism therein. The present inventors accordingly isolated the substance, studied its physical, chemical and biological characteristics and, based on these characteristics, confirmed that this substance which potentiates the antibacterial activity is a novel active substance. They termed it "Substance F2 which Potentiates the Activity of Antibiotics".

2

# 0 083 375

It should first be noted that in this specification the substance F2 which potentiates the activity of antibiotics will sometimes be referred to briefly as F2.

In the present invention the F2-producing strains are *Pseudomonas acidophila* G-6302 and *Pseudomonas mesoacidophila* SB-72310.

The strain G-6302 and the strain SB-72310 have been deposited at the Fermentation Research Institute, the Agency of Industrial Science and Technology (FRI, 1—3, Yatabecho higashi 1-chome, Tsukuba-gun, Ibaraki, Japan), Institute for Fermentation, Osaka (IFO, 17—85, Jusohonmachi 2-chome, Yodogawa-ku, Osaka, Japan) and The American Type Culture Collection (ATCC, 12301 Parklawn Drive, Rockville, Maryland 20852 U.S.A.), respectively, as follows:

| | | FRI | IFO | ATCC |
|---|---|---|---|---|
| The strain G-6302 | Deposit number | FERM-P No. 4344 | IFO 13774 | ATCC 31363 |
| | Date of deposit | The 20th day of December<br><br>The 52nd year of Showa (1977) | The 20th day of December<br><br>The 52nd year of Showa (1977) | December 20, 1977 |
| The strain SB-72310 | Deposit number | FERM-P No. 4653 | IFO 13884 | ATCC 31433 |
| | Date of deposit | The 13rd day of September<br><br>The 53rd year of Showa (1978) | The 13rd day of September<br><br>The 53rd year of Showa (1978) | September 27, 1978 |

The morphological characteristics of the strain G-6302 and the strain SB-72310 are described in U.S. Patent 4,229,436 (patented on October 21, 1980) and U.S. Patent 4,225,586 (patented on September 30, 1980), respectively.

In cultivating the above strains, there are employed such assimilable carbon sources as glucose, sucrose, maltose, spent molasses, glycerol, oils (e.g. soybean oil, olive oil.), organic acids (e.g. citric acid, succinic acid, gluconic acid). The nitrogen sources that may be employed include various organic and inorganic nitrogen compounds such as soybean meal, cottonseed meal, corn steep liquor, dried yeast, yeast extract, meat extract, peptone, urea, ammonium sulfate, ammonium nitrate, ammonium chloride, and ammonium phosphate. There are also employed such inorganic salts as are commonly used in the cultivation of bacteria, such as sodium chloride, calcium chloride, calcium carbonate, magnesium sulfate, monopotassium phosphate, and disodium phosphate. It was found that sulfur compounds which the strains can utilize, i.e. inorganic sulfur compounds such as sodium sulfate, and sodium thiosulfate, and organic sulfur compounds such as cystine, cysteine, and methionine, would lead to an increased yield of F2. Particularly preferred are cysteine and sodium thiosulfate. The concentration of such sulfur compounds is 0.01 to 1.0 w/v % and preferably 0.02 to 0.5 w/v %. The addition of a sulfur compound to the medium results in an increased yield of F2 and is therefore very advantageous in commercial-scale production.

If necessary, there are also incorporated in the medium heavy metal compounds such as ferrous sulfate, and tin sulfate, and vitamins such as vitamin $B_1$, and biotin. It is also possible or advantageous to add an antifoam or/and a surfactant, such as silicone oil, and polyalkylene glycol ether. In addition, organic or/and inorganic materials that will promote production of F2 may also be incorporated in suitable amounts.

The cultivation can be carried out either in solid medium or in liquid medium by a conventional antibiotics production method. While the cultivation in liquid medium may be conducted under standing still or with stirring, shaking or aeration, spinner culture under aeration is especially preferred. The cultivation is performed preferably at a temperature within the range of 15°C—35°C, at a pH of the medium within the range of 4—8, generally for 8—168 hours, preferably for 24—144 hours.

The thus-produced substance F2, which is present for the most part in the culture broth filtrate, is advantageously isolated and purified from the supernatant following separation of the culture broth into a supernatant and bacterial cells by centrifugation or filtration. It is also possible to isolate and purify substance F2 directly from the culture broth.

The potency of substance F2 can be determined, for instance, by the cup method or paper disc method using bouillon agar containing 0.05 µg/ml of cefmenoxime with *Escherichia coli* IFO 12734 as the test organism.

3

The substance F2 which potentiates the activity of antibiotics can be harvested by any means commonly used for harvesting metabolites produced by microorganisms. Thus, for example, bacterial cells are removed by centrifugation and the filtrate is subjected to a common process known for isolation and purification of effective substances. Thus, there are utilized solubility and solubility difference in an adequate solvent, difference in manner and rate of precipitation from a solution, difference in affinity with a variety of adsorbents, ion exchange chromatography with an ion exchanger, concentration under reduced pressure, lyophilization, crystallization, recrystallization, drying and other means, either alone or in combination in an optional order, either singly or repeatedly.

An example is as follows. The culture broth after completion of the cultivation is filtered, the filtrate is passed through an activated carbon column, and the thus-adsorbed substance F2 is eluted with a hydrophilic organic solvent system. The hydrophilic organic solvent system is, for example, a mixed solution composed of water and one or more of lower ketones, such as acetone, methyl ethyl ketone and methyl isobutyl ketone, and lower alcohols, such as methanol, ethanol, isopropanol, propanol and butanol. Since the desired substance is an acidic substance, the ion exchange resin is advantageously a Cl-type one, such as an anionic anion exchange resin (Amberlite® IRA-400, 402, Rohm & Haas, USA; Dowex®-1, Dow Chemical, USA; Diaion® SA-21A, Mitsubishi Chemical, Japan). The thus-adsorbed active substance, F2, is eluted with an aqueous solution of sodium chloride, for instance. For desalting, the eluate is again subjected to activated carbon column chromatography. The eluate containing the active substance is concentrated, then acetone is added, and the precipitate is collected by filtration, washed with acetone or ether and dried to give a pale brown powder. For further purification of the powder, column chromatography with QAE Sephadex® (Pharmacia, Sweden) is advantageous. Thus, after washing QAE Sephadex® A-25 is washed with phosphate buffer (pH 6.6), an aqueous solution of the above powder is passed through the column for adsorption, and the column is washed with the same buffer and then with the buffer containing 0.5 w/v % of sodium chloride and eluted with the buffer containing 1.0% of sodium chloride. The active fractions are pooled, adjusted to pH 3.0 and again passed through an activated carbon column. After washing with water and with 20 v/v % methanol-water, elution is performed with aqueous acetone. The active fractions are concentrated under reduced pressure. Upon addition of acetone, there is obtained F2. This substance can form a metal or ammonium salt. The metal salt is, for example, the sodium, potassium or lithium salt.

The physicochemical properties of the monosodium salt of F2 as obtained in Example 1 are as follows:

1. Melting point:
   216—219°C (decomposition)
2. Appearance:
   Colorless crystalline powder
3. Elemental analysis (for the sample dried over phosphorus pentoxide at 40°C under reduced pressure for 6 hours):

| | |
|---|---|
| C | 32.11(%) |
| H | 5.38 |
| N | 7.27 |
| S | 10.67 |
| Na | 3.8 |

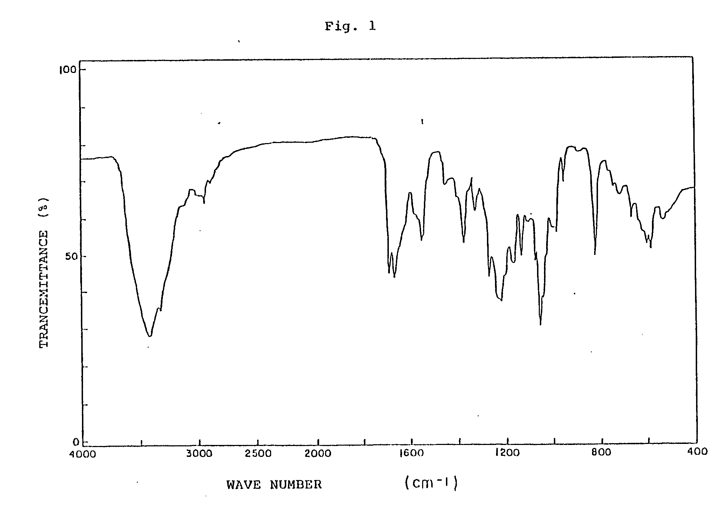

4. Molecular weight:
   605 (calculated as the monosodium salt); the molecular formula estimated from the above found values being $C_{15-16}H_{30-34}N_3O_{14-16}S_2Na$.
5. Ultraviolet absorption spectrum:
   Terminal absorption only (no characteristic absorption at wavelength over 210 nm)
6. Infrared absorption spectrum:
   An absorption spectrum as recorded by the potassium bromide disc method is shown in Fig. 1.
7. Specific rotation:
   $[\alpha]_D^{26}+6.85$ (c=0.38, N—CH$_3$COOH)
   $[\alpha]_D^{26}-7.72$ (c=0.544, pH 7 phosphate buffer)
8. Nuclear magnetic resonance spectrum (100 MHz, in dimethyl sulfoxide):
   A —CH$_3$ signal is observed at δ 1.84.
9. Solubility:
   Insoluble in petroleum ether, hexane, diethyl ether, benzene, ethyl acetate and chloroform; sparingly soluble in ethanol, pyridine and acetone; soluble in methanol and dimethyl sulfoxide; readily soluble in water.
10. Color reactions:
    Positive Greig-Leaback and potassium permanganate reactions; negative ninhydrin, Sakaguchi and Molisch reactions.
11. Stability:
    Stable in aqueous solution at pH 3—9 against heating at 60°C for 10 minutes.

12. Acidity/basicity:
    An acidic substance.

The physicochemical properties of the disodium salt of F2 as obtained in Example 2 are as follows:

1. Melting point:
   No distinct melting point
2. Appearance:
   White powder
3. Elemental analysis (for the sample dried over phosphorus pentoxide at 40°C under reduced pressure for 6 hours):

|   |   |
|---|---|
| C | 31.35(%) |
| H | 5.24 |
| N | 6.78 |
| S | 10.38 |
| Na | 7.1 |

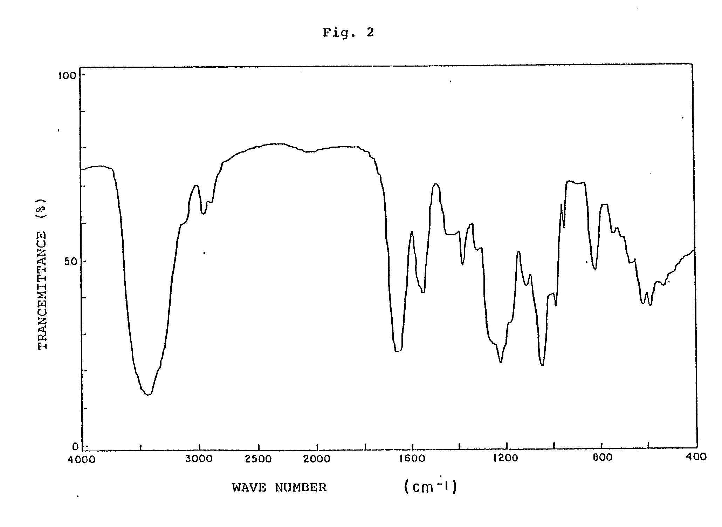

4. Molecular weight:
   647.6 on the supposition that each molecule contains 2 Na atoms.
5. Ultraviolet absorption spectrum:
   Terminal absorption only
6. Infrared absorption spectrum:
   An absorption spectrum as recorded by the potassium bromide disc method is shown in Fig. 2.
7. Specific rotation:
   $[\alpha]_D^{26} +5.5°$ (c=0.56, N—$CH_3COOH$)
8. Solubility:
   Insoluble in petroleum ether, hexane, diethyl ether, benzene, ethyl acetate, chloroform and acetone; sparingly soluble in methanol, ethanol and pyridine; soluble in dimethyl sulfoxide; readily soluble in water.
9. Color reactions:
   Positive Greig-Leaback and potassium permanganate reactions; negative ninhydrin, Sakaguchi and Molish reactions.
10. Stability:
    Stable in aqueous solution at pH 3—9 against heating at 60°C for 10 minutes.

The physico-chemical properties of the monosodium salt of F2 as obtained in Example 3 are as follows:

1. Melting point:
   207—208°C (decomposition)
2. Appearance:
   Colorless crystalline powder
3. Elemental analysis (for the sample dried over phosphorus pentoxide at 40°C under reduced pressure for 6 hours):

|   |   |
|---|---|
| C | 32.59(%) |
| H | 5.22 |
| N | 6.93 |
| S | 10.14 |
| Na | 3.7 |

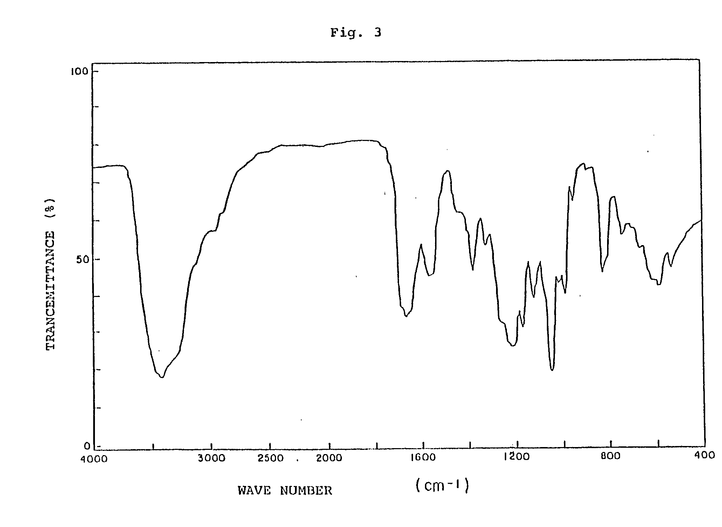

4. Molecular weight:
   621.6 (calculated as the monosodium salt); the molecular formula estimated from the above found values being $C_{15-16}H_{30-34}N_3O_{14-16}S_2 \cdot Na$.
5. Ultraviolet absorption spectrum:
   Terminal absorption only (no characteristic absorption at wavelengths over 210 nm)
6. Infrared absorption spectrum:
   An absorption spectrum as recorded by the potassium bromide disc method is shown in Fig. 3.
7. Specific rotation:
   $[\alpha]_D^{26} +6.0°$ (c=0.47, N—$CH_3COOH$)
8. Nuclear magnetic resonance spectrum (100 MHz, in dimethyl sulfoxide):
   A —$CH_3$ signal is observed at δ 1.84.
9. Solubility:
   Insoluble in petroleum ether, hexane, diethyl ether, benzene, ethyl acetate and chloroform; sparingly soluble in ethanol, pyridine and acetone; soluble in methanol and dimethyl sulfoxide; readily soluble in water.

10. Color reactions:
    Positive Greig-Leaback and potassium permanganate reactions; negative ninhydrin, Sakaguchi and Molisch reactions.
11. Stability:
    Stable in aqueous solution at pH 3—9 against heating at 60°C for 10 minutes.
12. Acidity/basicity:
    An acidic substance.

The physico-chemical properties of the disodium salt of F2 as obtained in Example 4 are as follows:

1. Melting point:
   No distinct melting point
2. Appearance:
   White powder
3. Elemental analysis (for the sample dried over phosphorus pentoxide at 40°C under reduced pressure for 6 hours):

| | |
|---|---|
| C | 30.83(%) |
| H | 5.35 |
| N | 6.91 |
| S | 9.68 |
| Na | 7.3 |

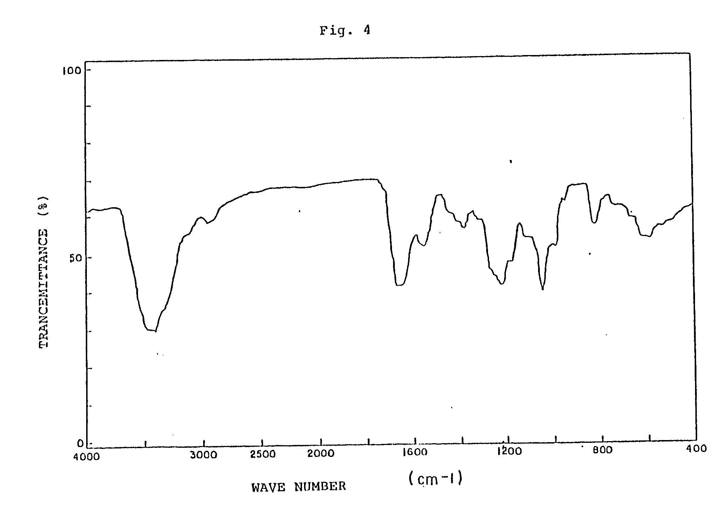

4. Molecular weight:
   629.9 on the supposition that each molecule contains 2 Na atoms.
5. Ultraviolet absorption spectrum:
   Terminal absorption only.
6. Infrared absorption spectrum:
   An absorption spectrum as obtained by the potassium bromide disc method is shown in Fig. 4.
7. Specific rotation:
   $[\alpha]_D^{26}+6.1°$ (c=0.39, N—$CH_3COOH$)
8. Solubility:
   Insoluble in petroleum ether, hexane, diethyl ether, benzene, ethyl acetate, chloroform and acetone; sparingly soluble in methanol, ethanol and pyridine; soluble in dimethyl sulfoxide; readily soluble in water.
9. Color reactions:
   Positive Greig-Leaback and potassium permanganate reactions; negative ninhydrin, Sakaguchi and Molisch reactions.
10. Stability:
    Stable in aqueous solution at pH 3—9 against heating at 60°C for 10 minutes.

As regards the biological properties of the substance F2 which potentiates the activity of antibiotics, it is evident from the data given in Table 1 and Table 2 that F2 exhibits an antibacterial activity against *Escherichia coli* in the copresence of cefmenoxime or mecillinam in a sub-bactericidal concentration.

Table 1: Cefmenoxime- and mecillinam-potentiating activity data for the F2 sample obtained in Example 1 (test organism: *Escherichia coli* IFO 12734)

TABLE 1

| F2 concentration in test solution** (µg/ml) | Diameter of growth inhibition zone (mm)* | | |
|---|---|---|---|
| | No addition | 0.05 µg/ml cefmenoxime | 0.03 µg/ml mecillinam |
| 0 | <8 | <8 | <8 |
| 1 | <8 | 10 | <8 |
| 5 | <8 | 15 | 10 |
| 25 | <8 | 24 | 18 |
| 125 | <8 | 32 | 24 |
| 1000 | <8 | 40 | 30 |

* The test was performed using a bouillon agar medium containing the antibiotic as specified and a paper disc, 8 mm in diameter.

** A paper disc, 8 mm in diameter, was impregnated with 25 µl of the test solution and placed on the agar medium

Table 2: Cefmenoxime- and mecillinam-potentiating activity data for the F2 sample obtained in Example 3 (test organism: *Escherichia coli* IFO 12734)

TABLE 2

| F2 concentration in test solution** (µg/ml) | Diameter of growth inhibition zone (mm)* | | |
|---|---|---|---|
| | No addition | 0.05 µg/ml cefmenoxime | 0.03 µg/ml mecillinam |
| 0 | <8 | <8 | <8 |
| 1 | <8 | 9 | <8 |
| 5 | <8 | 14 | 9 |
| 25 | <8 | 23 | 19 |
| 125 | <8 | 31 | 23 |
| 1000 | <8 | 39 | 29 |

* and **: The same as noted for Table 1.

It is also evident from the data given in Table 3 and Table 4 that F2 is capable of potentiating the antibacterial activity of cefmenoxime and mecillinam against *Proteus mirabilis* ATCC 21100.

Table 3: Cefmenoxime- and mecillinam-potentiating activity data for the F2 sample obtained in Example 1 (test organism: *Proteus mirabilis* ATCC 21100)*

TABLE 3

| F2 concentration in test solution (µg/ml) | Diameter of growth inhibition zone (mm)* | | |
|---|---|---|---|
| | No addition | 0.01 µg/ml cefmenoxime | 0.05 µg/ml mecillinam |
| 0 | <8 | <8 | <8 |
| 1 | <8 | 9 | 9 |
| 5 | <8 | 13 | 11 |
| 25 | <8 | 23 | 19 |
| 125 | <8 | 30 | 26 |
| 1000 | <8 | 38 | 32 |

* The test was performed under the same conditions as noted for Table 1 except for the test organism and the concentration of each coexisting antibiotic.

Table 4: Cefmenoxime- and mecillinam-potentiating activity data for the F2 sample obtained in Example 3 (test organism: *Proteus mirabilis* ATCC 21100)*

TABLE 4

| F2 concentration in test solution (µg/ml) | Diameter of growth inhibition zone (mm)* | | |
|---|---|---|---|
| | No addition | 0.01 µg/ml cefmenoxime | 0.05 µg/ml mecillinam |
| 0 | <8 | <8 | <8 |
| 1 | <8 | 9 | 9 |
| 5 | <8 | 12 | 12 |
| 25 | <8 | 22 | 19 |
| 125 | <8 | 29 | 25 |
| 1000 | <8 | 38 | 32 |

* The test was performed under the same conditions as noted for Table 1 except for the test organism and the concentration of each coexisting antibiotic.

Furthermore, F2 strongly inhibits the growth of *Escherichia coli* in synergy with cephalexin or cefmenoxime. In Table 5 and Table 6, there are shown degrees of growth of *Escherichia coli* under the influence of F2 in the copresence of 5 µg/ml of cephalexin or 1 µg/ml of cefmenoxime.

Table 5: Inhibition of growth of *Escherichia coli* IFO 12734 in the copresence of F2 (obtained in Example 1) and cephalexin or cefmenoxime.

TABLE 5

| Final F2 concentration (µg/ml) | Absorbance at 600 nm | | |
|---|---|---|---|
| | No addition | 5 µg/ml cephalexin | 1 µg/ml cefmenoxime |
| 0 | 2.6 | 2.35 | 1.95 |
| 1 | 2.65 | 1.80 | 0.60 |
| 10 | 2.65 | 1.00 | 0.25 |
| 100 | 2.70 | 0.95 | 0.30 |

To 8 ml of YAB medium (1.75% Difco antibiotic medium No. 3, 0.5% Difco yeast extract) containing 12% (w/v) of sucrose, there was added 1 ml of water, 50 µg/ml cephalexin solution or 10 µg/ml cefmenoxime solution, and the medium was inoculated with 1 ml of a preculture in YAB medium at an early logarithmic growth phase and incubated with shaking at 37°C for 2 hours. Thereafter, the absorbance of the culture broth was measured at 600 nm using a Shimadzu-Bausch & Lomb Spectronic® 20 colorimeter.

Table 6: Inhibition of growth of *Escherichia coli* IFO 12734 in the copresence of F2 (obtained in Example 3) and cephalexin or cefmenoxime*

TABLE 6

| Final F2 concentration (µg/ml) | Absorbance at 600 nm | | |
|---|---|---|---|
| | No addition | 5 µg/ml cephalexin | 1 µg/ml cefmenoxime |
| 0 | 2.4 | 2.15 | 1.90 |
| 1 | 2.45 | 1.75 | 0.55 |
| 10 | 2.50 | 1.90 | 0.25 |
| 100 | 2.55 | 0.75 | 0.25 |

* The test was performed in the same manner as noted for Table 5.

As is evident from Table 5 and Table 6, F2 inhibits the growth of *Escherichia coli* in the presence of cephalexin or cefmenoxime, as evidenced by the decrease in absorbance. Observation of the culture broths under a phase contrast microscope revealed that, when F2 was not added, cells were in the much elongated form under the influence of cephalexin or cefmenoxime but that, when F2 was added, the elongation was inhibited in dependence on the addition level and at the same time partial swelling occurred and bacteriolysis proceeded.

The substance F2 which potentiates the activity of antibiotics obtainable by the method of the present invention showed a marked antibacterial activity in synergy with cefmenoxime or cephalexin in *Escherichia coli-* or *Proteus mirabilis*-infected mice. Therefore, F2 can be used in combination with such antibiotics in the treatment of infectious diseases caused by the above bacteria in mammals (e.g. mouse, rat, dog, human) and in domestic fowls (e.g. chicken, duck).

For the treatment of *Escherichia coli* infection, for instance, a solution of 0.5—2.0 g of, for example, cefmenoxime and 0.5—10 g of F2 in 10—100 ml of physiological saline is administered to human adults two to three times a day in admixture with an injectable solution for intravenous drip infusion.

F2 is also a very promising intermediate for the synthesis of novel drugs.

When F2 was intravenously administered to mice, the $LD_{50}$ was more than 1 g/kg.

For comparison of the substance F2 which potentiates the activity of antibiotics, which has the above properties, there may be mentioned first of all such water-soluble, S-containing, acidic antibiotics as penicillins and cephalosporins. In contrast with these, substance F2 shows no absorption at wavelengths over 1720 cm$^{-1}$ in the infrared absorption spectrum and, when tested in a usual manner, does not show any antibacterial activity. Therefore, F2 is different from said antibiotics.

While there are known a number of antibiotics which are produced by microorganisms belonging to the genus *Pseudomonas,* a water-soluble, S-containing, acidic antibacterial antibiotic has not been known at all to be produced by a *Pseudomonas* species. Furthermore, F2 differs in physico-chemical and biological properties from any known antibiotics produced by other microorganisms than those belonging to the genus *Pseudomonas.* Therefore, it is concluded that the substance of the present invention is a novel compound.

Brief description of the drawings

Fig. 1, Fig. 2, Fig. 3 and Fig. 4 show infrared absorption spectra of the monosodium salt of F2 as obtained in Example 1, the disodium salt of F2 as obtained in Example 2, the monosodium salt of F2 as obtained in Example 3 and the disodium salt of F2 as obtained in Example 4, respectively.

Best mode for carrying out the invention

The following examples illustrate the invention in more detail. However, they should by no means be construed as limiting the present invention. Unless otherwise stated, each percentage value is on an weight/volume basis.

Example 1

Two 2-liter Sakaguchi flasks each containing 500 ml of a medium containing 1% glucose, 0.5% Polypepton® (Daigo Nutritive Chemicals, Ltd.), 0.5% meat extract and 0.5% sodium chloride (pH 7.0) were inoculated with cells of *Pseudomonas acidophila* G-6302 strain (FERM-P No. 4344; IFO 13774; ATCC 31363) grown on a slant nutrient agar and incubated at 28°C with reciprocal shaking for 48 hours to give a seed culture.

A 200-liter stainless steel fermenter was charged with 120 liters of a medium containing 3% glycerol, 0.1% glucose, 0.5% Polypepton®, 0.5% meat extract, 0.5% NaCl and 0.1% cysteine, and the medium was adjusted to pH 7.0 with 30% sodium hydroxide solution, then steam-sterilized at 120°C for 20 minutes, and inoculated with the above seed culture. Incubation was carried out at a temperature of 28°C, a rate of aeration of 120 liters/minute and a rate of stirring of 180 rpm for 78 hours. The culture broth was centrifuged on a Sharples centrifuge for separation of bacterial cells. There was obtained 110 liters of supernatant, which, after adjustment to pH 4.2, was passed through a column packed with 15 liters of activated carbon ("Shirasagi" for chromatographic use, Takeda Chemical Industries, Ltd.) for adsorption of the active substance. After washing with 45 liters of water, elution was conducted with 45 liters of 50 v/v% acetone-water. An aliquot of each 10-liter eluate fraction was tested for the active substance using a bouillon agar medium containing 0.05 µg/ml of cefmenoxime with *Escherichia coli* IFO 12734 as the test organism. As a result, fractions Nos. 2 and 3 were pooled and, after addition of 20 liters of water, passed through a column packed with 10 liters of Dowex®-1 (Cl) (Dow Chemical, USA). After washing with 25 liters of water, elution was carried out with 50 liters of 5% sodium chloride solution in water. The active fractions were pooled and, after adjustment to pH 4.0, passed through a column packed with activated carbon (8 liters). After washing with 24 liters of water, elution was performed with 20 v/v% methanol-water. The active fractions were concentrated under reduced pressure to 50 ml, 200 ml of acetone was added, and the resulting precipitate was collected by filtration, washed with 50 ml of acetone and 100 ml of ether and dried under reduced pressure to give 25 g of crude F2.

In 500 ml of M/25 phosphate buffer (pH 6.6) was dissolved 10 g of the crude F2 and the solution was passed through a 200-ml column of QAE Sephadex® A-25 (Pharmacia, Sweden) buffered with the same buffer as above, for adsorption of F2. The column was washed with 400 ml of the same buffer and then with 400 ml of the same buffer with 0.5% of sodium chloride added thereto. Thereafter, elution was carried out with the same buffer with 1% of sodium chloride added thereto. The active fractions were pooled and, after adjustment to pH 3.0 with 1N hydrochloric acid, passed through a 60-ml column of activated carbon. The column was washed with 200 ml of water and then with 100 ml of 50 v/v% methanol-water. Elution was carried out with 80 v/v% isobutanol-water. The active eluate fractions were pooled and, after adjustment to pH 5.5, concentrated under reduced pressure. The concentrate was dissolved by addition of 50 ml of methanol, and the solution was filtered and allowed to stand in a cool place. The resulting crystalline precipitate was filtered off, washed with ether and dried over phosphorus pentoxide at 40°C under reduced pressure for 6 hours. There was thus obtained 2.8 g of the monosodium salt of F2 as crystals. Its infrared absorption spectrum is shown in Fig. 1. Elemental analysis: C, 32.11; H, 5.38; N, 7.27, S, 10.67; Na, 3.8%

Example 2

In 90 ml of water was dissolved 3.0 g of the monosodium salt of F2 obtained in Example 1. After 4.5 ml of 1 N sodium hydroxide was added thereto under cooling, the solution was carefully adjusted to pH 7.0 with 1 N sodium hydroxide while measuring the pH and then was lyophilized to give 3.1 g of the disodium salt of F2 as a white powder. An infrared absorption spectrum for this product after drying at 40°C under reduced pressure for 6 hours is shown in Fig. 2. Elemental analysis gave the following results: C, 31.35; H, 5.24; N, 6.78; S, 10.38; Na, 7.1%.

Example 3

Two 2-liter Sakaguchi flasks each containing 500 ml of a medium containing 1% glucose, 0.5% Polypepton® (Daigo Nutritive Chemicals, Ltd.), 0.5% meat extract and 0.5% sodium chloride (pH 7.0) were inoculated with cells of *Pseudomonas mesoacidophila* SB-72310 (FERM-P No. 4653; IFO 13884; ATCC 31433) grown on a slant nutrient agar and incubated at 28°C with reciprocal shaking for 48 hours to give a seed culture.

A 200-liter stainless steel fermenter was charged with 120 liters of a medium containing 3% glycerol, 0.1% glucose, 0.5% Polypepton®, 0.5% meat extract, 0.5% NaCl and 0.1% cysteine, and the medium was adjusted to pH 7.0 with 30% sodium hydroxide solution, then steam-sterilized at 120°C for 20 minutes and inoculated with the above seed culture. Incubation was then carried out at a temperature of 28°C, a rate of aeration of 120 liters per minute and a rate of stirring of 180 rpm for 78 hours. The culture broth was centrifuged on a Sharples centrifuge for separation of bacterial cells. There was obtained 110 liters of supernatant, which, after adjustment to pH 4.2, was passed through a column packed with 15 liters of activated carbon ("Shirasagi" for chromatography, Takeda Chemical Industries, Ltd.) for adsorption of the active substance. After washing with 45 liters of water, elution was performed with 45 liters of 50 v/v% acetone-water. An aliquot of each 10-liter eluate fraction was tested for the active substance using a bouillon agar medium containing 0.05 µg/ml of cefmenoxime with *Escherichia coli* IFO 12734 as the test organism. Fractions Nos. 2 and 3 were pooled and, after addition of 20 liters of water, passed through a column packed with 10 liters of Dowex®-1 (Cl) (Dow Chemical, USA). After washing with 25 liters of water, elution was performed with 50 liters of 5% sodium chloride solution in water. The active fractions were pooled, adjusted to pH 4.0 and again passed through an activated carbon column (8 liters). Following washing with 24 liters of water, elution was carried out with 20 v/v% methanol-water. The active fractions were pooled and concentrated to 50 ml under reduced pressure, 200 ml of acetone added, and the resulting precipitate was collected by filtration, washed with 50 ml of acetone and 100 ml of ether and dried under reduced pressure. There was obtained 21 g of crude product.

In 500 ml of M/100 phosphate buffer (pH 6.6) was dissolved 10 g of the crude product and the solution was passed through a 200-ml column of QAE Sephadex® A-25 (Pharmacia, Sweden) buffered with the same buffer as above, for adsorption of F2. The column was washed with 400 ml of the same buffer and then with 400 ml of the same buffer with 0.5% of sodium chloride added thereto. Then, elution was performed with the same buffer with 1% of sodium chloride added thereto. The active fractions were pooled, adjusted, to pH 3.0 with 1 N hydrochloric acid and passed through a 60-ml column of activated carbon. The column was washed with 200 ml of water and elution was carried out with 8 v/v% isobutanol-water. The active fractions were pooled, adjusted to pH 5.5 and concentrated under reduced pressure. The concentrate was dissolved by addition of 50 ml of methanol and the solution was allowed to stand in a cool place. The resulting crystalline precipitates were collected by filtration, washed with ether and dried over phosphorus pentoxide at 40°C under reduced pressure for 6 hours. There was obtained 1.5 g of the monosodium salt of F2 as crystals. Its infrared absorption spectrum is shown in Fig. 3. Elemental analysis: C, 32.59; H, 5.22; N, 6.93; S, 10.14; Na, 3.7%.

Example 4

In 30 ml of water was dissolved 1.0 g of the monosodium salt of F2 as obtained in Example 3. Following addition of about 1.5 ml of 1 N sodium hydroxide under cooling, the solution was carefully adjusted to pH 7.0 while measuring the pH. The thus-neutralized solution was lyophilized to give 1.05 g of the disodium salt of F2 as a white powder. An infrared spectrum of this product after drying at 40°C for 6 hours is shown in Fig. 4. Elemental analysis gave the following data: C, 30.83; H, 5.35; N, 6.91; S, 9.68; Na, 7.3%.

Industrial applicability

A substance F2 which potentiates the activity of antibiotics potentiates the antibacterial activity of β-lactam antibiotics, and can be used in combination with such antibiotics in the treatment of infectious diseases caused by bacteria in mammals such as mouse, rat, dog, human and in domestic fowls such as chicken and duck.

**Claims**

1. A substance F2 which potentiates the activity of antibiotics against gram-negative bacteria, inclusive of salts thereof and salts produced synthetically therefrom, which as a monosodium salt thereof has the following physical and chemical properties:

(1) Appearance:
Colorless crystalline powder
(2) Elemental analysis (%):

| | |
|---|---|
| C | 31.81±1.0 |
| H | 5.35±0.5 |
| N | 6.96±0.5 |
| S | 10.81±1.0 |
| Na | 3.87±0.5 |

# 0 083 375

(3) Ultraviolet absorption spectrum:
No characteristic absorption at wavelength over 210 nm.

(4) Infrared absorption spectrum:
Main absorptions (wave numbers) in the IR region of the spectrum (KBr disc): 1690, 1665, 1555, 1375, 1220, 1050, 820 $(cm^{-1})$.

(5) Specific rotation:
$[\alpha]_D^{26}+6°\pm2°$ $(N—CH_3COOH)$

(6) Solubility:
Insoluble in petroleum ether, hexane, diethyl ether, benzene, ethyl acetate and chloroform; sparingly soluble in ethanol, pyridine and acetone; soluble in methanol and dimethyl sulfoxide; readily soluble in water.

(7) Basic, neutral or acidic:
Acidic

(8) Molecular weight:
594$\pm$77 (as monosodium salt, calcd. from sodium content)

(9) Color reactions:
Positive Greig-Leaback and potassium permanganate reactions; negative ninhydrin, Sakaguchi and Molisch reactions.

2. A method of producing a substance F2 as defined in Claim 1, characterised by cultivating in a culture medium the strain *Pseudomonas acidophila* (FERM-P 4344, IFO 13774, ATCC 31363) or *Pseudomonas mesoacidophila* (FERM-P 4653, IFO 13884, ATCC 31433) which is capable of producing the substance F2, and harvesting from the resulting culture broth the substance F2 thus produced as a metabolite.

3. The use of a substance F2 as defined in Claim 1 in association with a β-lactam antibiotic to make a therapeutic composition for the treatment of infectious diseases caused by gram-negative bacteria in mammals.

**Patentansprüche**

1. Substanz F2, die die Aktivität von Antibiotika gegen gramnegative Bakterien potenziert, einschließlich ihrer Salze und der synthetisch daraus hergestellten Salze, die als Mononatrium-Salz die folgenden physikalischen und chemischen Eigenschaften besitzt:

(1) Aussehen:
Farbloses kristallines Pulver

(2) Elementaranalyse (%):

| | |
|---|---|
| C | 31,81$\pm$1,0 |
| H | 5,35$\pm$0,5 |
| N | 6,96$\pm$0,5 |
| S | 10,81$\pm$1,0 |
| Na | 3,87$\pm$0,5 |

(3) Ultraviolett-Absorptionsspektrum:
Keine charakteristische Absorption bei Wellenlängen oberhalb von 210 nm.

(4) Infrarot-Absorptionsspektrum:
Haupt-Absorptionen (Wellenzahlen) im IR-Gebiet des Spektrums (KBr-Preßling): 1690, 1665, 1555, 1375, 1220, 1050, 820 $(cm^{-1})$.

(5) Spezifische Drehung:
$[\alpha]_D^{26}+6°\pm2°$ $(N—CH_3COOH)$

(6) Löslichkeit:
Unlöslich in Petrolether, Hexan, Diethylether, Benzol, Ethylacetat und Chloroform; wenig löslich in Ethanol, Pyridin und Aceton; löslich in Methanol und Dimethylsulfoxid; leicht löslich in Wasser.

(7) Basisch, neutral oder sauer:
Sauer

(8) Molekulargewicht:
594$\pm$77 (als Mononnatriumsalz, berechnet aus dem Natrium-Gehalt)

(9) Farbreaktionen:
Positive Greig-Leaback- und Kaliumpermanganat-Reaktionen; negative Ninhydrin-, Sakaguchi- und Molisch-Reaktionen.

2. Verfahren zur Herstellung einer Substanz F2, wie in Anspruch 1 definiert, gekennzeichnet durch Kultivieren des Stammes Pseudomonas acidophilia (FERM-P 4344, IFO 13774, ATCC 31363) oder Pseudomonas mesoacidophila (FERM-P 4653, IFO 13884, ATCC 31433), der zur Erzeugung der Substanz F2 befähigt ist, in einem Kulturmedium und Ernten der so als Metabolit erzeugten Substanz F2 aus der erhaltenen Kulturbrühe.

**0 083 375**

3. Verwendung einer Substanz F2, wie in Anspruch 1 definiert, in Verbindung mit einem β-Lactam-Antibiotikum für der Herstellung einer therapeutischen Zusammensetzung zur Behandlung von durch gramnegative Bakterien verursachten Infektionskrankheiten bei Säugern.

## Revendications

1. Substance F2 qui accroît l'effet des antibiotiques sur les bactéries gram-négatives, y compris les sels de cette substance et les sels de provenance synthétique de celle-ci, qui a, en tant que sel monosodique, les propriétés physiques et chimiques suivantes:

(1) Aspect:
poudre cristalline incolore
(2) Analyse élémentaire (%):

| | |
|---|---|
| C | $31,81\pm1,0$ |
| H | $5,35\pm0,5$ |
| N | $6,96\pm0,5$ |
| S | $10,81\pm1,0$ |
| Na | $3,87\pm0,5$ |

(3) Spectre d'absorption ultraviolet:
Point d'absorption caractéristique à une longueur d'onde supérieure à 210 nm.
(4) Spectre d'absorption infrarouge:
Absorptions principales (nombre d'ondes) dans la région IR du spectre (tablette de KBr): 1690, 1665, 1555, 1375, 1220, 1050, 820 ($cm^{-1}$).
(5) Pouvoir rotatoire spécifique:
$[\alpha]_D^{26} +6°\pm2°$ (N—$CH_3COOH$)
(6) Solubilité:
Insoluble dans l'éther de pétrole, l'hexane, l'éther diéthylique, le benzène, l'acétate d'éthyle et le chloroforme; faiblement soluble dans l'éthanol, la pyridine et l'acétone; soluble dans le méthanol et le diméthylsulfoxyde; très soluble dans l'eau.
(7) Basique, neutre ou acide:
Acide
(8) Poids moléculaire:
$594\pm77$ (sous forme de sel monosodique, calculé à partir de la teneur en sodium)
(9) Réactions colorées:
Réaction positive de Greig-Leaback et réaction positive au permanganate de potassium; réaction négative à la ninhydrine, réactions négatives de Sakaguchi et de Molisch.

2. Procédé de préparation de la substance F2 telle que définie à la revendication 1, caractérisé en ce que l'on cultive dans un milieu de culture la souche *Pseudomonas acidophila* (FERM-P 4344, IFO 13774, ATCC 31363) ou *Pseudomonas mesoacidophila* (FERM-P 4653, IFO 13884, ATCC 31433) qui est apte à produire la substance F2, et l'on recueille la substance F2 produite comme métabolite à partir du bouillon de culture résultant.

3. Utilisation de la substance F2 telle que définie à la revendication 1 en association avec un antibiotique β-lactamique, pour préparer une composition thérapeutique destinée au traitement des maladies infectieuses causées chez les mammifères par les bactéries gram-négatives.

Fig. 1

Fig. 2

Fig. 3

Fig. 4

0 083 375